# EUROPÄISCHE PATENTANMELDUNG

(11) **EP 4 675 268 A1**
(43) Veröffentlichungstag der Anmeldung: **07.01.2026**
(21) Anmeldenummer: 25186833.7
(22) Anmeldetag: 02.07.2025
(51) Int. Cl.: G01N 27/08, B01D 61/24, F16L 19/00

(54) **DIALYSE-LEITFÄHIGKEITSSONDE**

(30) Priorität: 04.07.2024 DE 102024118988
(71) Anmelder: B. Braun Avitum AG, 34212 Melsungen (DE)
(72) Erfinder: SCHWENDICH, Wilhelm, 36100 Petersberg (DE); BERTRAM, Rolf, 34286 Bergheim (DE)
(74) Vertreter: Winter, Brandl - Partnerschaft mbB

(57) **Zusammenfassung**

Offenbart ist eine Dialyse-Leitfähigkeitssonde mit einem Stapel, der aus mindestens zwei Leitfähigkeitsmesszellen (2) und aus mindestens einem Abstandhalter (4) dichtend zusammengesetzt ist, wobei die Anzahl der Leitfähigkeitsmesszellen (2) um eins größer als die Anzahl des Abstandhalters (4)/ der Abstandhalter (4) ist. Der Stapel ist zwischen einem ersten Abschlussteil (6a) und einem zweiten Abschlussteil (6b) dichtend eingespannt. Die Leitfähigkeitsmesszellen (2) und der mindestens eine Abstandhalter (4) und die Abschlussteile (6a, 6b) haben jeweils einen zentralen Kanalabschnitt (1, 3, 5), aus denen ein durchgehender Messkanal der Dialyse-Leitfähigkeitsmesssonde zusammengesetzt ist. Der Stapel ist mittels Zugstangen (10; 110) zwischen den beiden Abschlussteilen (6a, 6b) eingespannt, wobei die Zugstangen (10; 110) am Außenumfang des Stapels verteilt sind. Ein jeweiliger erster Endabschnitt (12a)jeder Zugstange (10; 110) erstreckt sich durch ein jeweiliges Durchgangsloch (14a) des ersten Abschlussteils (6a), während ein jeweiliger zweiter Endabschnitt (12b) jeder Zugstange (10; 110) sich durch ein jeweiliges Durchgangsloch (14b) des zweiten Abschlussteils (6b) erstreckt. An den Endabschnitten (12a) der Zugstangen (10; 110) ist eine jeweilige vorzugsweise umlaufende Nut (16a, 16b) oder ein jeweiliger Einstich vorgesehen, in die oder in den ein jeweiliges Sicherungselement (18a, 18b) eingesetzt ist, das an eine äußere Nutflanke (17a, 17b) der jeweiligen Nut (16a, 16b) gespannt ist.

## Beschreibung

### Technisches Gebiet

Die vorliegende Offenbarung betrifft eine Dialyse-Leitfähigkeitssonde, die zur Leitfähigkeitsmessung bei der Herstellung von Dialysierflüssigkeit in Dialysegeräten eingesetzt wird.

### Hintergrund der Offenbarung

Dialyse-Leitfähigkeitssonden werden zur Leitfähigkeitsmessung bei der Herstellung von Dialysierflüssigkeit eingesetzt. Dazu werden in der Dialyse-Leitfähigkeitssonde mehrerer Leitfähigkeitsmesszellen mittels mehrerer Abstandhalter zueinander beabstandet angeordnet und so ein Stapel gebildet, bei dem Kanalabschnitte der Leitfähigkeitsmesszellen zusammen mit den dazwischen angeordneten Kanalabschnitten der Abstandhalter einen Messkanal ausbilden. Dieser Messkanal wird während der Herstellung bzw. Aufbereitung der Dialysierflüssigkeit von deren Komponenten Permeat und Bikarbonat-Konzentrat oder danach von der fertigen Dialysierflüssigkeit durchströmt.

Die Leitfähigkeitsmesszellen und die Abstandhalter des Stapels sind in üblicher Weise dichtend aneinandergepresst, damit der Messkanal keine Undichtigkeiten bei den Übergängen zwischen den genannten Komponenten aufweist. Darüberhinausgehend ist an beiden Endabschnitten des Stapels ein jeweiliges Abschlussteil, das auch als Flansch bezeichnet werden kann, vorgesehen. An den beiden Abschlussteilen sind auch der Eingangsanschluss bzw. der Ausgangsanschluss der Dialyse-Leitfähigkeitssonde gebildet. Auch diese Abschlussteile sind in üblicher Weise dichtend an die erste bzw. letzte Leitfähigkeitsmesszelle gespannt.

### Stand der Technik

Aus dem Stand der Technik ist es bekannt, gleichmäßig am Umfang des Stapels verteilt vier Zuganker bzw. Zugstangen vorzusehen, die den Stapel und jeweilige Druckfedern einspannen. Mit Schraubverbindungen werden die Federkräfte bzw. Spannkräfte eingestellt. Dazu werden die Zugstangen beidseitig mit einem metrischen Gewinde versehen und einseitig in das untere Abschlussteil (in den unteren Flansch) eingeschraubt. In dem unteren Abschlussteil ist kein Gewinde vorgeformt, und die Gewindestage muss sich ein Gewinde in dem Abschlussteil selbst formen. Das metrische Gewinde ist aber für solche Verschraubungen nicht ausgelegt. In dem oberen Bereich, in dem auch die Druckfedern angeordnet sind, werden Muttern auf die jeweilige Gewindestange aufgeschraubt. Dort kommt es immer wieder dazu, dass die Muttern die Gewindestangen durch Fressen beschädigen. Dies führt zu Schwankungen im Verschraubungsprozess und muss immer wieder korrigiert werden. Die dichtende Einspannung per Verschraubung ist also nicht ausreichend prozesssicher. Auch nimmt das Aufschrauben der Muttern mit Umdrehungszählung zu viel Zeit in Anspruch.

### Kurzbeschreibung der Offenbarung

Aufgabe der vorliegenden Offenbarung ist es, eine Dialyse-Leitfähigkeitssonde zu schaffen, deren Produktion, insbesondere deren finales dichtendes Zusammenpressen der Komponenten des Stapels vereinfacht und prozesssicherer ist.

Diese Aufgabe wird mit der Merkmalskombination des Anspruchs 1 gelöst. Vorteilhafte Ausführungsformen und Weiterbildungen sind in den abhängigen Ansprüchen beansprucht und/oder werden nachfolgend erläutert.

Die Dialyse-Leitfähigkeitssonde gemäß der Offenbarung hat einen Stapel, der aus mindestens zwei Leitfähigkeitsmesszellen und aus mindestens einem Abstandhalter in abwechselnder Reihenfolge, das heißt in der Reihenfolge Leitfähigkeitsmesszelle, Abstandhalter, Leitfähigkeitsmesszelle, ggfls. weiterer Abstandhalter, weitere Leitfähigkeitsmesszelle, ggfls. usw., zusammengesetzt (gestapelt) ist. Vorzugsweise sind mehrere Leitfähigkeitsmesszellen und mehrere Abstandhalter vorgesehen. Die Anzahl der Leitfähigkeitsmesszellen ist insbesondere um eins größer als die Anzahl des Abstandhalters/ der Abstandhalter. Der Stapel ist zwischen einem ersten Abschlussteil und einem zweiten Abschlussteil eingespannt. Die Abschlussteile können auch als Flansch bezeichnet werden. Die Leitfähigkeitsmesszellen und der mindestens eine Abstandhalter und die beiden Abschlussteile haben jeweils einen zentralen Kanalabschnitt, aus denen ein durchgehender Messkanal der Dialyse-Leitfähigkeits(mess)-sonde zusammengesetzt ist. Daher werden insbesondere die genannten Komponenten dichtend aneinandergedrückt. Der Stapel ist dazu mittels mindestens dreier Zugstangen, vorzugsweise vier Zugstangen, zwischen den beiden Abschlussteilen eingespannt. Die Zugstangen sind am Außenumfang des Stapels, vorzugsweise gleichmäßig, verteilt. Ein erster Endabschnitt jeder Zugstange erstreckt sich durch ein jeweiliges Durchgangsloch des ersten Abschlussteils, während sich ein jeweiliger zweiter Endabschnitt durch ein jeweiliges Durchgangsloch des zweiten Abschlussteils erstreckt. Gemäß der Offenbarung ist am ersten Endabschnitt jeder Zugstange eine jeweilige erste, vorzugsweise umlaufende, Nut oder ein Einstich vorgesehen, in die/ den ein jeweiliges erstes Sicherungselement eingesetzt ist, das von dem Stapel an eine äußere Nutflanke der jeweiligen ersten Nut gespannt ist. Unter der äußeren Nutflanke ist die sich weiter entfernt von dem Stapel befindliche Nutflanke der beiden Nutflanken der Nut bzw. des Einstichs zu verstehen. Auf gleiche Weise ist am zweiten Endabschnitt jeder Zugstange eine jeweilige zweite, vorzugsweise umlaufende, Nut oder ein Einstich vorgesehen, in die/ den ein jeweiliges zweites Sicherungselement eingesetzt ist, das von dem Stapel an eine äußere Nutflanke der jeweiligen zweiten Nut gespannt ist. Auch hier ist unter der äußeren Nutflanke die sich weiter entfernt von dem Stapel befindliche Nutflanke der beiden Nutflanken der Nut bzw. des Einstichs zu verstehen. Damit ist das dichtende Zusammenpressen der Komponenten des Stapels mit den beiden Abschlussteilen vereinfacht und prozesssicher. Offenbarungsgemäß müssen insbesondere keine Gewinde auf die Zugstangen geschnitten werden. Es entfällt in vorteilhafter Weise das Einschrauben der Gewindestangen in den Kunststoff des zweiten Abschlussteils. Ein Fressen der Muttern auf den Gewinden der ersten Endabschnitte der Zugstangen ist aufgrund des Nichtvorhandenseins von Gewinden an den Zugstangen ausgeschlossen. Ein Abstand der beiden Nuten bzw. Einstiche jeder Zugstange, insbesondere ein Abstand der beiden äußeren Nutflanken kann präzise bemessen werden und genau auf einen geforderten Temperaturbereich abgestimmt werden, in dem die Dialyse-Leitfähigkeitssonde betrieben werden soll.

Vorzugsweise sind vier Zugstangen gleichmäßig am Außenumfang des Stapels verteilt. Dann können zwei einander (diagonal) gegenüberliegende Zugstangen ohne Kontakt zu den Außenumfängen der Komponenten des Stapels (Leitfähigkeitsmesszellen und Abstandhalter) sein, während die beiden anderen einander (diagonal) gegenüberliegende Zugstangen in Halte- und/oder Führungsvorrichtungen der Abstandhalter eintauchen, die sich radial nach außen in Richtung zu den beiden betroffenen Zugstangen erstrecken. Offenbarungsgemäß ist jedoch auch eine andere Anzahl von Zugstangen, etwa drei, fünf, sechs, sieben, etc. denkbar und es ist auch denkbar, dass mehr als zwei Zugstangen, beispielsweise drei, vier, fünf, sechs, sieben, etc. in Halte- und/oder Führungsvorrichtungen der Abstandhalter eintauchen.

Die Sicherungselemente können allseits bzw. vollumfänglich geschlossen sein und axial entlang einer Längsachse der Zugstange bis zur Nut aufgeschoben sein. Bei den Verschraubungen des Standes der Technik ist die präzise Positionierung der Muttern an den jeweiligen Gewinden der ersten Endabschnitte der Zugstangen schwierig. Diesbezüglich bilden die offenbarungsgemäßen Nuten bzw. Einstiche und insbesondere nicht elastische Sicherungselemente große Vorteile. Wenig Elastizität ermöglichen Sicherungselemente, die seitlich offen sind, und die in radialer Richtung zur Längsachse der Zugstange auf die Nut aufgeschoben sind. Das heißt offenbarungsgemäß hat sich insbesondere herausgestellt, dass Sicherungselemente, welche seitlich offen sind und in radialer Richtung zur Längsachse der Zugstange auf die Nut bzw. den Einstich aufgeschoben werden, vorteilhaft im Vergleich zu vollumfänglich geschlossenen und axial entlang der Längsachse der Zugstange bis zur Nut/ zu dem Einstich aufgeschobenen Sicherungselementen sind. Als bevorzugte Sicherungselemente haben sich dabei Sicherungsscheiben für Wellen (Wellensicherungsscheiben) oder SEEGER-Ringe herausgestellt. Besonders bevorzugte Sicherungselemente sind dabei Sicherungsscheiben für Wellen. Derartige Sicherungsscheiben können aufgrund ihrer Bauform radial auf der Zugstange montiert werden, indem sie einfach ohne Spezialwerkzeug in die Nut bzw. den Einstich hineingedrückt werden. Es hat sich offenbarungsgemäß herausgestellt, dass derartige Sicherungsscheiben für die auftretenden eher niedrigen Axialbelastungen geeignet sind.

Optimal präzise ist die Stapel zusammen mit den beiden Abschussteilen eingespannt, wenn die äußeren Nutflanken gemäß einer bevorzugten Weiterbildung senkrecht zur Längsachse der betroffenen Zugstange sind.

Um eine Elastizität des Stapels zu erzeugen, können elastische Dichtringe genutzt werden, die zwischen den Leitfähigkeitsmesszellen und den benachbarten Abstandhaltern und zwischen den Leitfähigkeitsmesszellen und den benachbarten Abschlussteilen angeordnet sind.

Um die angesprochene Elastizität des Stapels zu erzeugen, werden bevorzugt an den ersten Endabschnitten der Zugstangen jeweilige Druckfedern angeordnet, die zwischen dem jeweiligen ersten Sicherungselement und dem ersten Abschlussteil eingespannt sind.

Zwischen den Druckfedern und dem ersten Abschlussteil kann eine erste einteilige oder mehrteilige Kraftvergleichmäßigungsvorrichtung für die Federkräfte nötig sein. An den ersten Sicherungselementen kann innenseitig, also stapelseitig oder druckfederseitig eine zweite einteilige oder mehrteilige Kraftvergleichmäßigungsvorrichtung nötig sein. An den zweiten Sicherungselementen kann innenseitig, also stapelseitig eine dritte einteilige oder mehrteilige Kraftvergleichmäßigungsvorrichtung nötig sein.

Die mehrteiligen Kraftvergleichmäßigungsvorrichtung(en) können von kostengünstigen Unterlegscheiben gebildet sein. Die Anzahl der Unterlegscheiben pro Kraftvergleichmäßigungsvorrichtung entspricht dabei insbesondere der Anzahl der Zugstangen.

Die Kraftvergleichmäßigungsvorrichtung(en) kann/können einteilig ausgeführt sein und von einem jeweiligen Einlegeblech mit Durchgangslöchern gebildet sein. Die Anzahl der Durchgangslöcher pro Einlegeblech entspricht insbesondere der Anzahl der Zugstangen. Damit kann die Montage vereinfacht sein. Insbesondere bietet das zuerst montierte Einlegeblech eine Montagehilfe in Form einer Positionierhilfe.

Bei der Weiterbildung mit vier Zugstangen wird das Einlegeblech im Wesentlichen viereckig, insbesondere quadratisch, ausgeführt, wobei in jeder Ecke ein Durchgangsloch für eine der Zugstangen vorgesehen ist.

Wenn das Einlegeblech mindestens einen äußeren Steg oder eine äußere Nase hat, der oder die sich z.B. in radialer Richtung weg von der Längsachse einer benachbarten Zugstange erstreckt, dann kann das Einlegeblech von einer Lichtschranke detektiert werden und seine Anwesenheit sichergestellt werden.

Vorzugsweise sind acht Stege oder Nasen am Einlegeblech vorgesehen, wobei an jeder Ecke des Einlegeblechs zwei Stege oder Nasen vorgesehen sind, die vorzugsweise etwa rechtwinklig zueinander angeordnet sind. Damit ergibt sich maximale Freiheit bei der Montage der Einlegebleche, denn jedes Einlegeblech kann in acht verschiedenen Positionen angeordnet, also auf die vier Zugstangen geschoben werden.

### Kurzbeschreibung der Figuren

Figur 1 ist eine Dialyse-Leitfähigkeitssonde gemäß einem ersten Ausführungsbeispiel der vorliegenden Offenbarung in einer geschnittenen Darstellung;
Figur 2 ist die Dialyse-Leitfähigkeitssonde gemäß dem ersten Ausführungsbeispiel aus Figur 1 in einer anderen geschnittenen Darstellung;
Figur 3 ist eine Dialyse-Leitfähigkeitssonde gemäß einem zweiten Ausführungsbeispiel in einer perspektivischen Darstellung; und
Figur 4 ist eine Dialyse-Leitfähigkeitssonde gemäß einem dritten Ausführungsbeispiel in einer perspektivischen Darstellung.

### Beschreibung der Ausführungsbeispiele

Nachstehend werden drei Ausführungsbeispiele der vorliegenden Offenbarung auf der Basis der zugehörigen Figuren beschrieben.

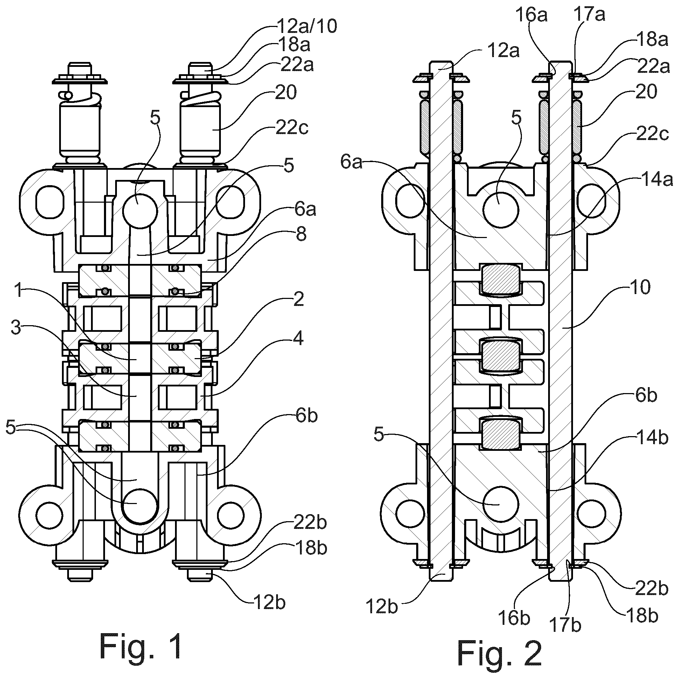

Figur 1 ist eine Dialyse-Leitfähigkeitssonde gemäß einem ersten Ausführungsbeispiel der vorliegenden Offenbarung in einem Schnitt entlang einem zentralen Messkanal, der aus Kanalabschnitten 1 von Leitfähigkeitsmesszellen 2 und aus Kanalabschnitten 3 von Abstandhaltern 4 und aus gekrümmten Kanalabschnitten 5 von Abschlussteilen 6a, 6b zusammengesetzt ist. Genauer gesagt weist die Dialyse-Leitfähigkeitssonde einen Stapel auf, der beim gezeigten ersten Ausführungsbeispiel aus drei Leitfähigkeitsmesszellen 2 und zwei dazwischengesetzten Abstandhaltern 4 besteht. Der Stapel wird (in Figur 1 oben und unten) von einem jeweiligen Abschlussteil 6a, 6b begrenzt, das auch als Flansch oder Anschlussteil bezeichnet werden kann, weil diese beiden Teile einen Eingang in den Messkanal und einen Ausgang aus dem Messkanal bereitstellen.

Die Leitfähigkeitsmesszellen 2 können auch als Elektroden bezeichnet werden und haben einen leitfähigen Innenmantel, der den jeweiligen Kanalabschnitt 1 bildet, und einen jeweiligen Hauptkörper der elektrisch nicht leitend ist. Die Abstandhalter 4 sind ebenfalls elektrisch nicht leitend und haben jeweils zwei Anlageflächen für die benachbarten Leitfähigkeitsmesszellen 2. Die Leitfähigkeitsmesszellen 2 haben einen puk-artigen Aufbau, von dem sich jeweils ein (nicht gezeigter) elektrischer Anschluss-Kontakt radial erstreckt.

Zwischen den genannten Komponenten (Leitfähigkeitsmesszellen 2, Abstandhalter 4 und Abschlussteile 6a, 6b) der Dialyse-Leitfähigkeitssonde sind jeweilige Dichtringe 8 vorgesehen, die dichtend aufeinandergepresst werden. Dazu erstrecken sich vier parallel zum Messkanal ausgerichtete Zugstangen 10 am Außenumfang des Stapels und durch die Abschlussteile 6a, 6b. Es sind in dieser Schnittdarstellung nur zwei Zugstangen 10 erkennbar, die im Wesentlichen verdeckt sind.

Figur 2 ist die Dialyse-Leitfähigkeitssonde gemäß dem ersten Ausführungsbeispiel aus Figur 1 in einer geschnittenen Darstellung, wobei die Schnittebene in zwei der insgesamt vier Zugstangen 10 liegt. Erste (in Figur 2 obere) Endabschnitte 12a jeder Zugstange 10 erstrecken sich durch ein jeweiliges Durchgangsloch 14a des ersten Abschlussteils 6a. Zweite (in Figur 2 untere) Endabschnitte 12b jeder Zugstange 10 erstrecken sich durch ein jeweiliges Durchgangsloch 14b des zweiten Abschlussteils 6b.

An den beiden Endabschnitten 12a, 12b jeder Zugstange 10 sind umlaufende Nuten 16a, 16b eingebacht, in die ein jeweiliges als einseitig offener Sicherungsring bzw. als einseitig offene Sicherungsscheibe ausgebildetes Sicherungselement 18a, 18b eingeschoben ist.

Zwischen dem (in Figur 2 oberen) ersten Sicherungselement 18a jeder Stange 10 und dem ersten Abschlussteil 6a ist eine jeweilige Druckfeder 20 angeordnet. Jede Druckfeder 20 stützt sich einerseits über eine Unterlegscheibe 22a an dem jeweiligen ersten Sicherungselement 18a und andererseits über eine weitere Unterlegscheibe 22c an dem ersten Abschlussteil 6a ab.

Zwischen dem (in Figur 2 unteren) zweiten Sicherungselement 18b jeder Stange 10 und dem zweiten Abschlussteil 6b ist eine Unterlegscheibe 22b angeordnet.

Ein Abstand der beiden Nuten 16a, 16b bzw. Einstiche jeder Zugstange 10, insbesondere ein Abstand der beiden äußeren Nutflanken 17a ,17b, gegen die der jeweilige Sicherungsring bzw. die jeweilige Sicherungsscheibe 18a, 18b gepresst wird, ist präzise gewählt und gefertigt und genau auf einen geforderten Temperaturbereich abgestimmt, in dem die Dialyse-Leitfähigkeitssonde betrieben werden soll.

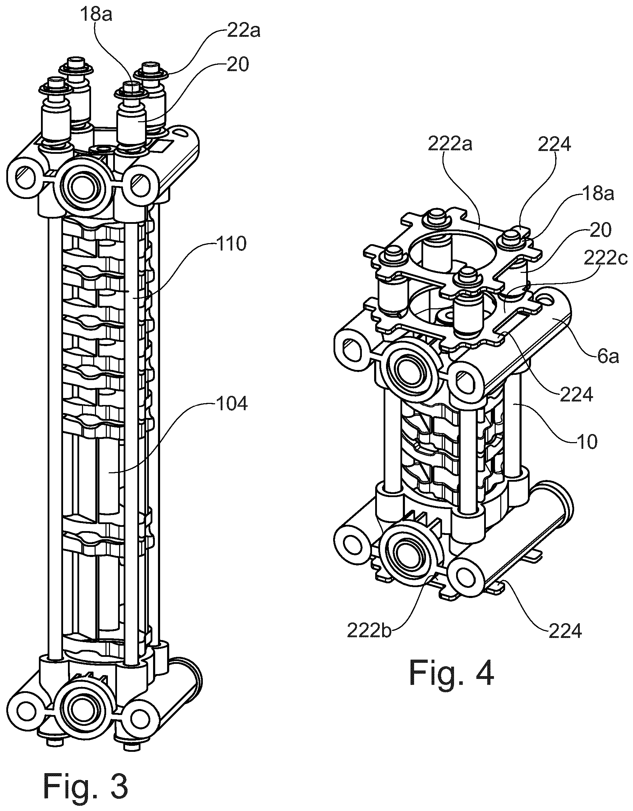

Figur 3 ist eine Dialyse-Leitfähigkeitssonde gemäß einem zweiten Ausführungsbeispiel in einer perspektivischen Darstellung. Die Unterschiede zum ersten Ausführungsbeispiel gemäß den Figuren 1 und 2 sind darin zu sehen, dass die vier Zugstangen 110 verlängert sind, sodass mehr Leitfähigkeitsmesszellen 2 und mehr Abstandhalter 4 und weiterhin auch vergrößerte Abstandhalter 104 angeordnet werden können.

Daraus ergibt sich, dass die Leitfähigkeitssonde der Figuren 1 und 2 als so genannte BIC-LF-Sonde eingesetzt wird, währen die Leitfähigkeitssonde der Figur 3 als so genannte END-LF-Sonde eingesetzt wird.

Bei der Dialyse-Leitfähigkeitssonde gemäß Figur 3 entspricht die Verspannungstechnik mit den Nuten 16a, 16b und den als Sicherungsringen bzw. Sicherungsscheiben ausgebildeten Sicherungselementen 18a, 18b und den aus jeweils vier Unterlegscheiben 22a, 22b gebildeten Kraftvergleichmäßigungsvorrichtungen derjenigen des ersten Ausführungsbeispiels aus den Figuren 1 und 2.

Figur 4 ist eine Dialyse-Leitfähigkeitssonde gemäß einem dritten Ausführungsbeispiel in einer perspektivischen Darstellung. Die Unterschiede zum ersten Ausführungsbeispiel gemäß den Figuren 1 und 2 sind darin zu sehen, dass die drei Kraftvergleichmäßigungsvorrichtungen nicht aus jeweils vier Unterlegscheiben gebildet sind, sondern dass jede der drei Kraftvergleichmäßigungsvorrichtungen von einem Einlegeblech 222a, 222b, 222c gebildet ist. Die Einlegebleche 222a, 222b, 222c sind im Wesentlichen quadratisch wobei in jeder Ecke ein Durchgangsloch für eine der Zugstangen 10 vorgesehen ist.

Von jeder Ecke der Einlegebleche 222a, 222b, 222c erstrecken sich zwei Stege 224 oder Nasen unter einem Winkel von 90 Grad zueinander weg vom Rand. Die beiden Stege 224 oder Nasen können sich dabei in radialer Richtung weg von dem in der betroffenen Ecke angeordneten Durchgangsloch und damit in radialer Richtung weg von der in der betroffenen Ecke angeordneten Zugstange 10 erstecken. Einer der insgesamt acht Stege 224 wird bei einer Montagekontrolle durch eine (nicht gezeigte) Lichtschranke erfasst und so die Anwesenheit des betroffenen Einlegeblechs 222a, 222b, 222c geprüft.

Durch die insgesamt acht Stege 224 ist maximale Freiheit bei der Montage gegeben, denn es sind insgesamt acht korrekte Montagepositionen jedes Einlegeblechs 222a, 222b, 222c möglich.

Bei der Dialyse-Leitfähigkeitssonde der Figur 4 entsprechen die Verspannungstechnik mit den Nuten 16a, 16b und den als Sicherungsringen bzw. Sicherungsscheiben ausgebildeten Sicherungselementen 18a, 18b und darüberhinausgehend auch der Stapel aus drei Leitfähigkeitsmesszellen 2 und zwei dazwischengesetzten Abstandhaltern 4 denjenigen des ersten Ausführungsbeispiels aus den Figuren 1 und 2.

Die vier Druckfedern 20 und die beiden Abschlussteile 6a, 6b aller gezeigten Ausführungsbeispiele sind gleich.

### Bezugszeichenliste

- 1: Kanalabschnitt (von 2)
- 2: Leitfähigkeitsmesszelle
- 3: Kanalabschnitt (von 4)
- 4: (kürzerer) Abstandhalter
- 5: gekrümmter Kanalabschnitt
- 6a: erstes Abschlussteil
- 6b: zweites Abschlussteil
- 8: Dichtring
- 10: (kürzere) Zugstange
- 12a: erster Endabschnitt
- 12b: zweiter Endabschnitt
- 14a: Durchgangsloch des ersten Abschlussteils
- 14b: Durchgangsloch des zweiten Abschlussteils
- 16a: erste Nut
- 16b: zweite Nut
- 17a: erste äußere Nutflanke
- 17b: zweite äußere Nutflanke
- 18a: erstes Sicherungselement
- 18b: zweites Sicherungselement
- 20: Druckfeder
- 22a: Unterlegscheibe
- 22b: Unterlegscheibe
- 22c: Unterlegscheibe
- 104: (längerer) Abstandhalter
- 110: (längere) Zugstange
- 222a: Einlegblech
- 222b: Einlegblech
- 222c: Einlegblech

## Patentansprüche

1. Dialyse-Leitfähigkeitssonde mit einem Stapel, der aus mindestens zwei Leitfähigkeitsmesszellen (2) und aus mindestens einem Abstandhalter (4) dichtend zusammengesetzt ist, wobei
die Anzahl der Leitfähigkeitsmesszellen (2) um eins größer als die Anzahl des Abstandhalters (4)/ der Abstandhalter (4) ist,
der Stapel zwischen einem ersten Abschlussteil (6a) und einem zweiten Abschlussteil (6b) dichtend eingespannt ist,
die Leitfähigkeitsmesszellen (2) und der mindestens eine Abstandhalter (4) und die Abschlussteile (6a, 6b) jeweils einen, insbesondere zentralen, Kanalabschnitt (1, 3, 5) haben, aus denen ein durchgehender Messkanal der Dialyse-Leitfähigkeitsmesssonde zusammengesetzt ist,
der Stapel mittels mindestens dreier Zugstangen (10; 110) zwischen den beiden Abschlussteilen (6a, 6b) eingespannt ist,
die Zugstangen (10; 110) am Außenumfang des Stapels verteilt sind, wobei erste Endabschnitte (12a) jeder Zugstange (10; 110) sich durch ein jeweiliges Durchgangsloch (14a) des ersten Abschlussteils (6a) erstrecken, und wobei zweite Endabschnitte (12b) jeder Zugstange (10; 110) sich durch ein jeweiliges Durchgangsloch (14b) des zweiten Abschlussteils (6b) erstrecken,
**dadurch gekennzeichnet, dass**
an den ersten Endabschnitten (12a) jeder Zugstange (10; 110) eine jeweilige erste Nut (16a) oder ein jeweiliger erster Einstich vorgesehen ist, in die oder in den ein jeweiliges erstes Sicherungselement (18a) eingesetzt ist, das an eine äußere Nutflanke (17a) der jeweiligen ersten Nut (16a) gespannt ist, und dass
an den zweiten Endabschnitten (12b) jeder Zugstange (10; 110) eine jeweilige zweite vorzugsweise umlaufende Nut (16b) oder ein jeweiliger zweiter Einstich vorgesehen ist, in die oder in den ein jeweiliges zweites Sicherungselement (18b) eingesetzt ist, das an eine äußere Nutflanke (17b) der jeweiligen zweiten Nut (16b) gespannt ist.

2. Leitfähigkeitssonde nach Anspruch 1, **dadurch gekennzeichnet, dass** die Sicherungselemente (18a, 18b) seitlich offen sind und radial zu einer Längsachse der Zugstange (10; 110) auf die Nut (16a, 16b) oder den Einstich aufgeschoben sind.

3. Leitfähigkeitssonde nach Anspruch 1 oder 2, **dadurch gekennzeichnet, dass** die äußeren Nutflanken (17a, 17b) senkrecht zu einer Längsachse der betroffenen Zugstange (10; 110) sind.

4. Leitfähigkeitssonde nach einem der vorhergehenden Ansprüche, **dadurch gekennzeichnet, dass** zwischen den Leitfähigkeitsmesszellen (2) und den benachbarten Abstandhaltern (4) und zwischen den Leitfähigkeitsmesszellen (2) und den benachbarten Abschlussteilen (6a, 6b) jeweils elastische Dichtringe (8) angeordnet sind.

5. Leitfähigkeitssonde nach einem der vorhergehenden Ansprüche, **dadurch gekennzeichnet, dass** an den ersten Endabschnitten (12a) der Zugstangen (10; 110) Druckfedern (20) angeordnet sind, die zwischen dem jeweiligen ersten Sicherungselement (18a) und dem ersten Abschlussteil (6a) eingespannt sind.

6. Leitfähigkeitssonde nach Anspruch 5, **dadurch gekennzeichnet, dass** zwischen den Druckfedern (20) und dem ersten Abschlussteil (6a) eine Kraftvergleichmäßigungsvorrichtung (22c; 222c) für die Federkräfte angeordnet ist.

7. Leitfähigkeitssonde nach einem der vorhergehenden Ansprüche, **dadurch gekennzeichnet, dass** an den ersten Sicherungselementen (18a) stapelseitig oder druckfederseitig eine erste Kraftvergleichmäßigungsvorrichtung (22a; 222a) angelegt ist, und/oder dass an den zweiten Sicherungselementen (18b) stapelseitig eine zweite Kraftvergleichmäßigungsvorrichtung (22b; 222b) angelegt ist.

8. Leitfähigkeitssonde nach Anspruch 6 und/oder 7, **dadurch gekennzeichnet, dass** die Kraftvergleichmäßigungsvorrichtung(en) von Unterlegscheiben (22a; 22b; 22c) gebildet ist/sind.

9. Leitfähigkeitssonde nach Anspruch 6 und/oder 7, **dadurch gekennzeichnet, dass** die Kraftvergleichmäßigungsvorrichtung(en) von einem jeweiligen Einlegeblech (222a; 222b; 222c) mit Durchgangslöchern gebildet ist/sind.

10. Leitfähigkeitssonde nach Anspruch 9, **dadurch gekennzeichnet, dass** vier Zugstangen (10) vorgesehen sind, und dass das Einlegeblech (222a; 222b; 222c) im Wesentlichen viereckig ist, wobei in jeder Ecke ein Durchgangsloch für eine der Zugstangen (10) vorgesehen ist.

11. Leitfähigkeitssonde nach Anspruch 9 oder 10, **dadurch gekennzeichnet, dass** das Einlegeblech (222a; 222b; 222c) mindestens einen äußeren Steg (224) oder eine äußere Nase hat, der oder die sich bevorzugt in radialer Richtung weg von der Längsachse einer der Zugstangen (10) erstreckt.

12. Leitfähigkeitssonde nach den Ansprüchen 10 und 11, **dadurch gekennzeichnet, dass** acht Stege oder Nasen vorgesehen sind, wobei an jeder Ecke des Einlegeblechs (222a; 222b; 222c) zwei Stege (224) oder Nasen vorgesehen sind, die vorzugsweise etwa rechtwinklig zueinander angeordnet sind.
